# EUROPEAN PATENT SPECIFICATION

(11) **EP 2 437 700 B1**
(45) Date of publication and mention of the grant of the patent: **26.06.2013**
(21) Application number: 10720216.0
(22) Date of filing: 07.05.2010
(51) Int. Cl.: A61F 9/007, A61B 18/08

(54) **CAPSULARHEXIS DEVICE WITH FLEXIBLE HEATING ELEMENT HAVING AN ANGLED TRANSITIONAL NECK**
INSTRUMENT FÜR CAPSULARHEXIS MIT FLEXIBLEM HEIZELEMENT MIT ABGEWINKELTEM ÜBERGANGSHALS
DISPOSITIF DE CAPSULO-RHEXIS A ELEMENT CHAUFFANT FLEXIBLE POURVU D'UN COL DE TRANSITION INCLINE

(30) Priority: 03.06.2009 US 477175
(43) Date of publication of application: 11.04.2012
(73) Proprietor: Alcon Research, Ltd., Fort Worth, Texas 76134 (US)
(72) Inventor: JIA, Guangyao, Irvine California 92612 (US); SUSSMAN, Glenn Robert, Laguna Niguel California 92677 (US)
(74) Representative: Hanratty, Catherine
(86) International application number: PCT/US2010/033949
(87) International publication number: WO 2010/141181

(56) References cited:
- WO-A1-99/60936
- US-A- 4 481 948
- US-A1- 2006 100 617
- US-A1- 2010 094 278
- US-B1- 6 551 326

## Description

### FIELD OF THE INVENTION

The present invention relates generally to the field of ophthalmic surgery and more particularly to an apparatus for performing a capsularhexis.

### DESCRIPTION OF THE RELATED ART

An accepted treatment for the treatment of cataracts is surgical removal of the lens (e.g., through phacoemulsification) and replacement of the lens function by an artificial intraocular lens (IOL). Prior to removing the cataractous lens, an opening, or rhexis, may be made in the anterior capsule. During phacoemulsification, there may be tension on the cut edges of the anterior capsularhexis while the lens nucleus is emulsified. Further, if the capsule is opened with numerous small capsular tears, the small tags that remain may lead to radial capsular tears that may extend into the posterior capsule. Such a radial tear may constitute a complication since it may destabilize the lens for further cataract removal and safe intraocular lens placement within the lens capsule later in the operation. In addition, if the posterior capsule is punctured then the vitreous may gain access to the anterior chamber of the eye. If this happens, the vitreous may need to be removed by an additional procedure with special instruments. The loss of vitreous may lead to subsequent retinal detachment and/or infection within the eye. Further, while some ophthalmic procedures may also require a posterior capsularhexis, current devices designed for anterior capsularhexis may not have an optimal geometry for performing a posterior capsularhexis.

### SUMMARY OF THE INVENTION

The embodiment includes a capsularhexis device with a resistive-heating element comprising an electrically resistive, superelastic wire forming a loop with a gap between first and second ends of the superelastic wire. The capsularhexis device may further include an insulating portion comprising an electrically insulating material separating the first and second ends of the superelastic wire. The insulating portion may be used to retract the loop into a collapsed, retracted position inside an insertion sleeve. The insulating portion may also be used to eject/expand the loop into an expanded position outside of the insertion sleeve. The first and second ends of the loop may be adjacent to each other and may at least partially extend at an angle from a planar face defined by the loop, to the insulating portion, to form a transitional neck between the loop and the insulating portion. A prior art device is shown in WO 99/60936. The transitional neck may have a gap between the first and second ends at the insulating portion that is wider than a gap between the first and second ends on the opposing side of the transitional neck. This gap may be sufficiently small to allow the loop to form a continuous cut in a capsule of an eye when current is applied to the loop while positioned in contact with the capsule. The loop may be used for anterior capsulotomy and/or posterior capsulotomy. If used for posterior capsulotomy, the loop may be circular and may have a diameter that is smaller than loops used for anterior capsulotomy. Other loop shapes and sizes are also contemplated.

The invention is defined in the claims.

### BRIEF DESCRIPTION OF THE DRAWINGS

For a more complete understanding of the present invention, reference is made to the following description taken in conjunction with the accompanying drawings in which:
FIGs. 1a-b illustrate various positions of a capsularhexis device, according to an embodiment;
FIGs. 1c-d illustrate a head-on, cross-sectional view of two embodiments of a transitional neck for a capsularhexis device;
FIGs. 1e-f illustrate an embodiment of the loop for posterior capsulorhexis.
FIGs. 1g-h illustrate an embodiment of the loop for anterior capsulorhexis.
FIGs. 2a-b illustrate an embodiment of the handpiece.
FIGs. 2c-d illustrate an embodiment of an exposed loop and a withdrawn loop.
FIGs. 3a-d illustrate expansion and retraction of the capsularhexis device through an insertion sleeve, according to an embodiment;
FIG. 4 illustrates an angled capsularhexis device, according to an embodiment;
FIG. 5 illustrates a side view of the capsularhexis device inserted into the posterior capsule, according to an embodiment;
FIGs. 6a-b illustrate alternate configurations of the wire used in the capsularhexis device, according to various embodiments;
FIG. 7 illustrates a flowchart of a method for performing a capsulotomy, according to an embodiment, the method not being part of the invention; and
FIG. 8 illustrates a processor and memory for the capsularhexis device.

It is to be understood that both the foregoing general description and the following detailed description are exemplary and explanatory only and are intended to provide a further explanation of the present invention as claimed.

### DETAILED DESCRIPTION OF THE EMBODIMENTS

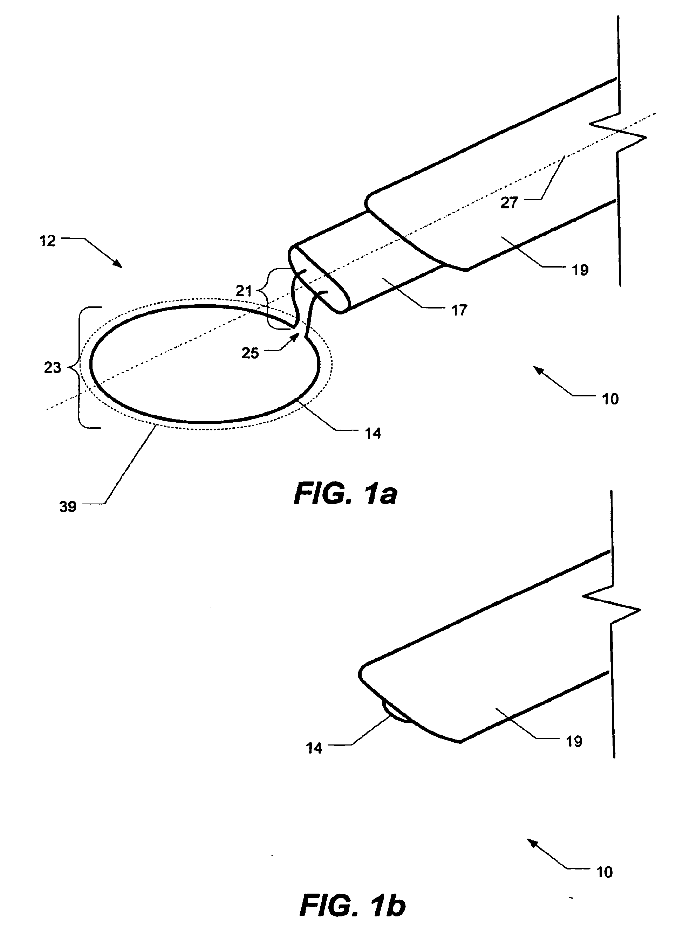

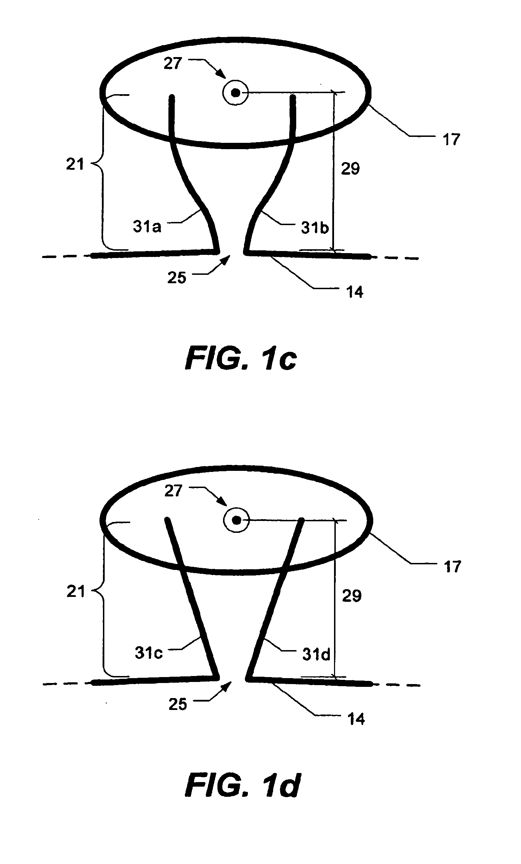

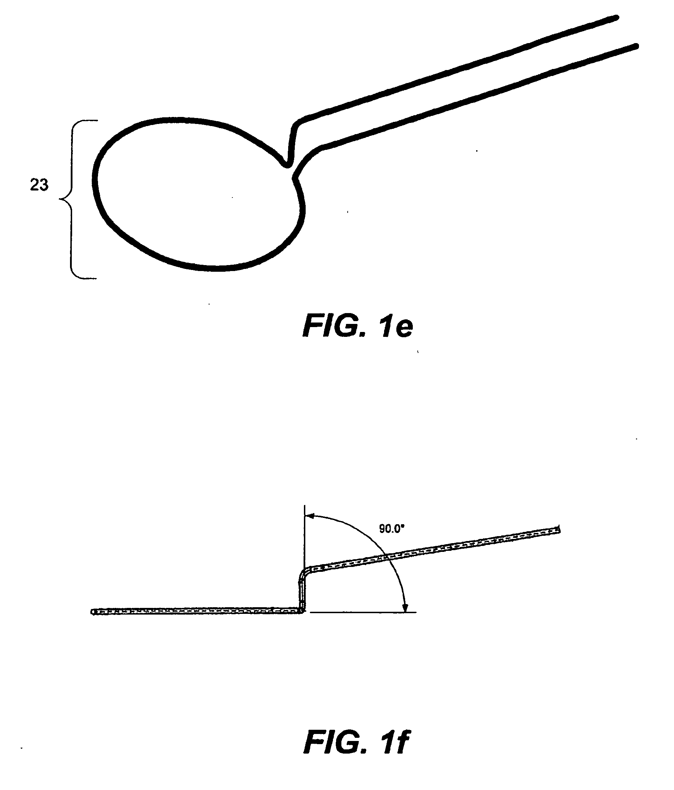

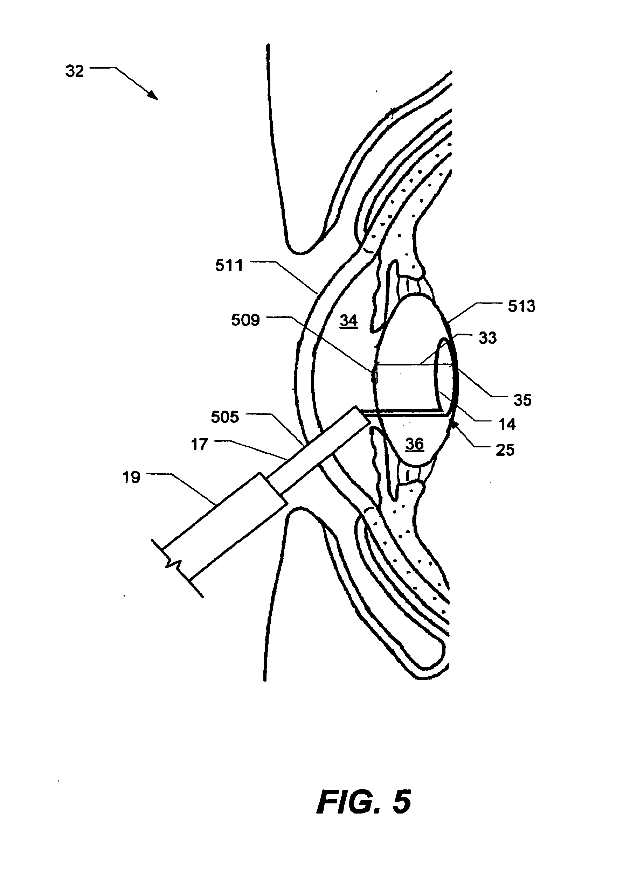

FIGs. 1a-b show a plan view of some embodiments of a capsularhexis device 10. Those skilled in the art will appreciate that FIGs. 1a-b, like the several other attached figures, are not to scale, and that several of the features may be exaggerated to more clearly illustrate various features. Those skilled in the art will also appreciate that the illustrated structures are only exemplary, and not limiting. The capsularhexis device 10 includes a substantially circular, flexible loop 23 of a resistance-heating element 12 that may be energized to produce localized heating on an anterior lens capsule 509 and/or posterior lens capsule 513 (e.g., see FIG. 5) of an eye 32 to create a through cut or define a weakened boundary for detachment of the portion of the capsule 36 within the loop 23. The capsularhexis device 10 may be positioned within the anterior chamber 34 through a small incision 505 to perform the capsularhexis, or capsulotomy. This procedure may facilitate, for example, phacoemulsification of a cataractous lens and insertion of an artificial intraocular lens (IOL).

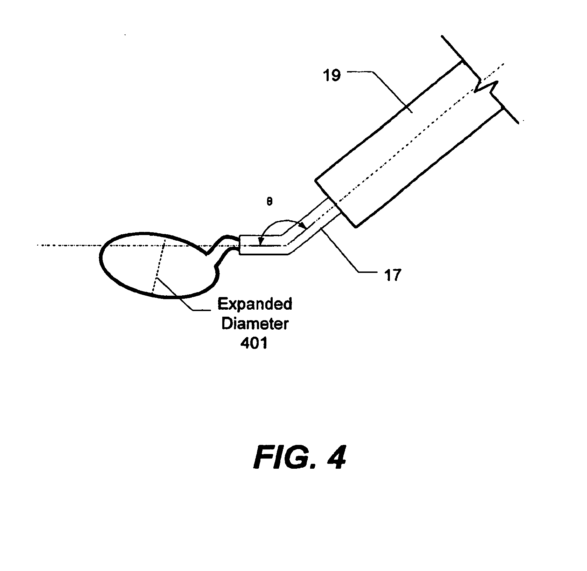

As seen in FIGs 1a-d, in various embodiments, the heating element 12 may include a transitional neck 21 (e.g., formed by first and second wire ends 31a-b or 31c-d (referred to generally herein as wire ends 31)) with an offsetting bend so as to offset a planar face 39 of the loop 23 above or below a centerline 27 of an insertion sleeve 19. The wire ends 31 forming transitional neck 21 may bend away from the centerline 27 (e.g., a distance 29 as shown in FIG. 1c). Bending away from the centerline 27 may allow the loop 23 to be placed more parallel with an anterior and/or posterior capsule face. As seen in FIG. 5, the wire ends 31 in the transitional neck 21 may displace the loop 23 a depth 33 of the capsule 36 to position the loop 23 for uniform contact with the posterior capsule face 35. Since the heat-affected zone of the wire 14 is smaller on the capsule because of the perpendicular orientation with respect to the capsule surface, thermal insulation may not be needed for prevention of collateral thermal damage to the capsule region underneath. In some embodiments, the diameter 401 (e.g., see FIG. 4) of the loop 23 may be adjusted according to whether the loop 23 will be used in anterior capsulorhexis or posterior capsulorhexis (which may use a smaller diameter 401 (e.g., approximately in a range of 2-4 millimeters (mm)) than in anterior capsulorhexis which may use a diameter approximately in a range of 4-6 mm). Other diameters are also contemplated. In some embodiments, the transitional neck 21 may have a length (a distance from the insulating portion 17 to the loop 23) of approximately 1-2 mm (other lengths are also contemplated).

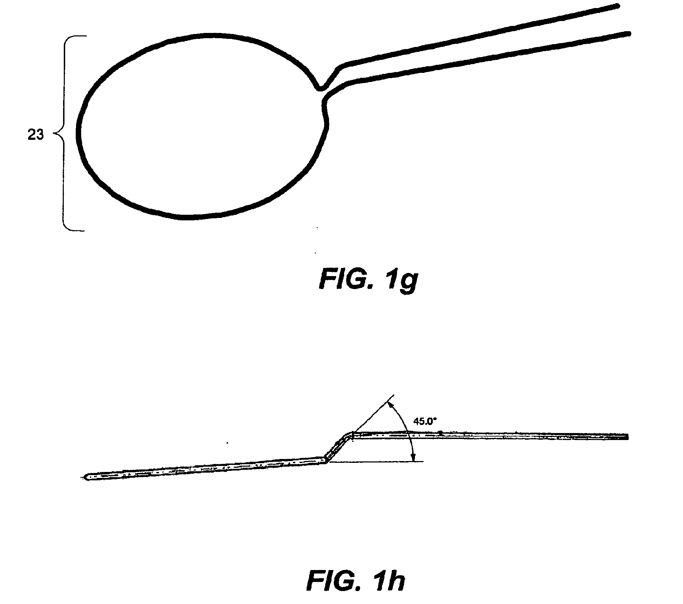

In some embodiments, the transitional neck 21 may be substantially (e.g., +/- 20 degrees) perpendicular to a planer face 39 of the loop 23 (e.g., for posterior capsulorhexis as seen in FIGs. 1e-f). Other angles are also contemplated. For example, the transitional neck 21 may be approximately 135 degrees (e.g., for anterior capsulorhexis as seen in FIGs. 1g-h) or 45 degrees measured to a back side of the plane as seen in FIG. 1h. Other angles are also contemplated (e.g., the transitional neck may be approximately in a range of 30 degrees to 90 degrees from a back side of the plane). In some embodiments, the wire ends 31 may be bent toward each other to reduce the size of gap 25 between the wire ends 31 of the resistance-heating element 12. The gap 25 may be minimized to maintain enough distance to prevent a short between ends of the gap (i.e., so current travels around the loop 23). For example, the gap 25 may have a width of approximately 0.003 inches plus or minus 0.001 inches. Other dimensions are also contemplated (e.g., 0.006 inches or, as another example, smaller than 0.002 inches). The gap 25 may insulate the wire ends 31 from each other (such that electric current travels through wire 14 and not across gap 25). Bending away from the centerline 27 may allow a further reduction in the size of gap 25 than would be otherwise possible if the wire ends 31 were parallel to the centerline 27. The reduced gap size may result in a more complete circular through cut or a boundary for detachment. (While a circular loop 23 is shown, other shapes are also contemplated (e.g., elliptical, rectangular, etc)). Due to the reduced gap size, contact with the capsule 36 and wire 14 around gap 25 may provide bipolar diathermy in the capsule 36 to facilitate a more complete capsulotomy despite the discontinuity (i.e., gap 25) on the heating element 12. The angled orientation of the transitional neck 21 with respect to the planar face 39 may reduce straight edges in the capsule 36 at the gap 25 to form a more circular ring with complete (or mostly complete) rhexis. Neighboring heat from the wire 14 on either side of the gap 25 may thermally cut the portion of the capsule 36 between the gap 25 because of the reduced width of gap 25.

Wire ends 31 may be curved and/or straight (see FIGs. 1c-d). Other configurations for the wire ends 31 are also contemplated. While the term "bending" is used throughout, the wire ends 31a-b may be formed and/or shaped using other methods (e.g., mold casting, extrusion, etc).

In various embodiments, the geometry of the loop 23 may be adjusted based on whether the loop 23 will be used for posterior capsulorhexis (e.g., see FIGs. 1e-f) or anterior capsulorhexis (e.g., see FIGs. 1g-h).

According to several embodiments, the resistive-heating element 12 may include an at least partially bare resistance-heating element made from a super-elastic wire. By combining the super-elasticity of the wire material with a relatively high electric resistivity, a collapsible, ring-shaped heating element 12 may be constructed to perform capsulotomy by localized heating. Because the heating element 12 may be collapsible, the heating element 12 may be easily inserted into the eye 32 through a small incision 505 (e.g., 2 mm) in the cornea 511. Other incision sizes and locations are also contemplated.

The capsularhexis device 10 may include a fine, superelastic wire 14 for the heating element 12. In some embodiments, the wire 14 may be formed from a nickel titanium alloy, such as Nitinol, which may exhibit superelastic and shape memory properties. Because the wire 14 may be superelastic (which term is intended herein as a synonym for the somewhat more technically precise term "pseudoelastic"), the wire 14 may be able to withstand a significant amount of deformation when a load is applied and return to its original shape when the load is removed. (Those skilled in the art will appreciate that this property is distinct from, although related to, "shape memory", which refers to a property exhibited by some materials in which an object that is deformed while below the material's transformation temperature returns to its former shape when warmed to above the transformation temperature. Nitinol exhibits both properties; superelasticity is exhibited above the transformation temperature.) Further, Nitinol is resistive, and can thus be heated with an electrical current, making it useful for forming the resistive-heating element 12 illustrated in FIGs. 1a-c. Of course, those skilled in the art will appreciate that other materials that are resistive and superelastic may be used instead of Nitinol in some embodiments.

Because the wire 14 has superelastic properties, the wire may be able to collapse during insertion and return to a pre-formed shape during use. A viscoelastic agent may be used to inflate the anterior chamber 34 prior to the capsulotomy. The viscoelastic agent may have a sufficiently low thermal diffusivity to serve as a thermal insulator around the heating element 12, thus facilitating the formation of a highly concentrated thermally affected zone in the immediate vicinity of the heating element 12. The concentration of this zone may reduce collateral damage to nearby tissue. Although in practice it may be unavoidable to trap a thin film of viscoelastic material between the heating element and the capsule, a small defined area on the capsule 36 may still respond sufficiently fast to the temperature rise in the heating element to avoid collateral damage, due to the small thickness (e.g., approximately 10 micrometers) of the fluid film.

The resistive-heating element 12 may include a loop 23 formed from the superelastic wire 14. The ends of the wire 14, extending away from the loop 23 to form a lead section, may be kept electrically separate with a flexible, electrically insulating portion 17. In some embodiments, the insulating portion 17 may surround a portion of the lead section. However, those skilled in the art will appreciate that insulating portion 17 may surround only one lead, or may only partially surround either or both leads, in some embodiments, provided that the two leads extending away from the loop 23 and into the insertion sleeve 19 may be kept electrically separate so that electrical current may be passed through the loop of the resistive-heating element 12. Insulating portion 17 may include a bio-compatible and high temperature-resistant material, such as polyimide or Teflon™. In some embodiments, insulating portion 17 may be flexible. In some embodiments, one or more crimp tubes (e.g., silver crimp tubes) may be used to receive the loop 23 (the tubes may be crimped onto the loop 23 to secure the loop 23 into the handpiece). In some embodiments, insulating portion 17 may extend over the crimp tubes to electrically insulate the tubes from each other.

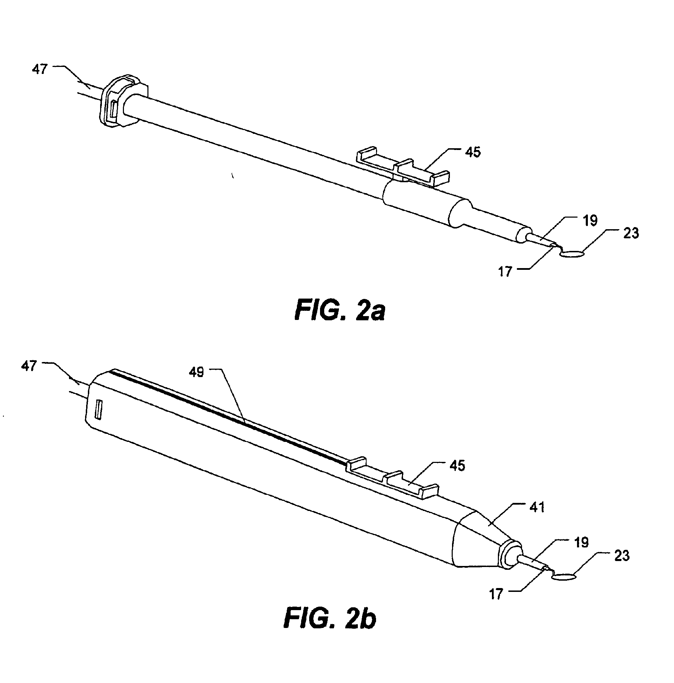

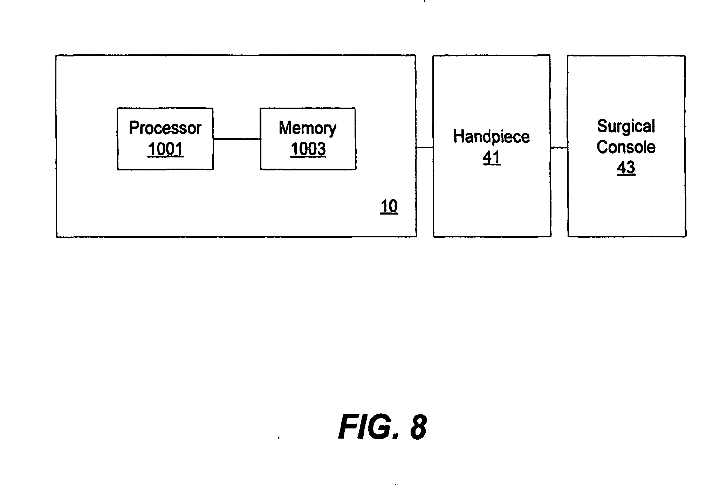

In some embodiments, insertion sleeve 19 may include a flat or cylindrical tube that engages a portion of a lead section, including the insulating portion 17. In some embodiments, the insertion sleeve 19 may form a slip-fit with the insulating portion 17. Insertion sleeve 19 may be used to insert the heating element 12 into the eye 32 during the capsularhexis procedure and to retract the heating element 12 afterwards. The insertion sleeve 19, which may be made from a thermoplastic, may also contain electrical connectors and/or connecting wires so that the heating element 12 may be selectively connected to a power source for heating. In some embodiments, the insertion sleeve 19, insulation material 17, and wire 14 may form a disposable unit that can be selectively connected during use to a handpiece or other apparatus that can supply electrical current. In some embodiments, insertion sleeve 19 may be coupled to handpiece 41 (e.g., see FIGs. 2a-b) which may be coupled to a surgical console 43 (e.g., see FIG. 8).

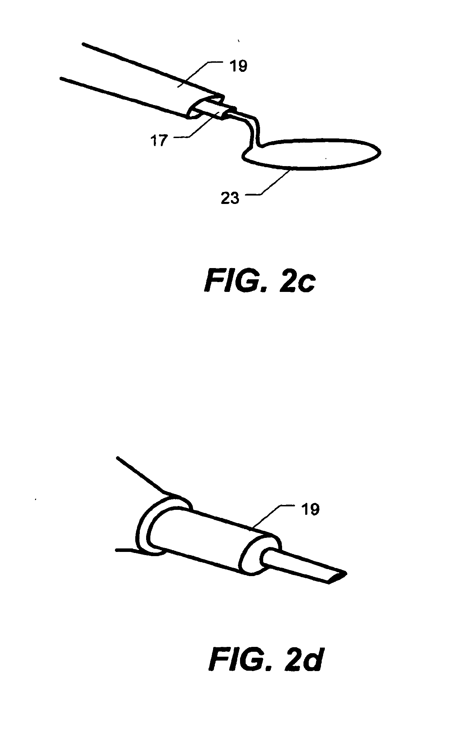

Because of its superelastic properties, the heating element 12 may be collapsed for insertion into the anterior chamber 34 of the eye 32, regaining its pre-defined shape within the anterior chamber 34. Accordingly, some embodiments include or may be used with an insertion sleeve 19 through which the heating element 12 is pushed. A collapsed heating element 12 in a retracted position in the insertion sleeve 19 is shown in FIG. 1b and FIG. 2d. The heating element 12 may be collapsible upon retracting the heating element 12 into the insertion sleeve 19 and expandable to its original shape upon ejection from the insertion sleeve 19. In some embodiments, the insertion sleeve 19 and insulating portion 17 may be incorporated in a single device (or separate devices). In some embodiments, a separate cartridge may be used to collapse/expand the loop 23 through (e.g., separate from and/or in place of insertion sleeve 19). As seen in FIGs. 2a-b, a handpiece 41 may include a retraction lever 45 which may ride in a slot 49. When retraction lever 45 (attached to the insertion sleeve) is pushed towards the end of the slot 49, the loop 23 may be enclosed in the insertion sleeve 19 (e.g., see FIG. 2d). When the retraction lever 45 is pulled back along the slot 49, the loop 23 may exit the insertion sleeve 19 (see FIG. 2c). Other configurations of the handpiece are also contemplated. In various embodiments, the loop 23 may be partially withdrawn into the insertion sleeve 19 (e.g., as seen in FIG. 1b) or fully withdrawn into the insertion sleeve 19 (e.g., as seen in FIG. 2d) before and/or after the procedure. In some embodiments, the partially exposed wire (as seen in FIG. 1b) may act as a guide as the insertion sleeve 19 is inserted into an incision.

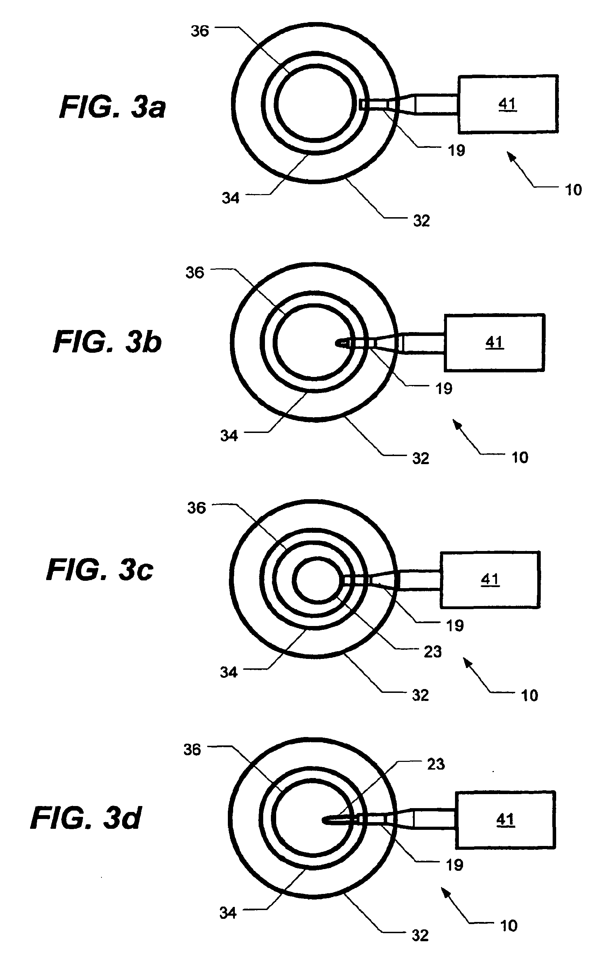

FIGs. 3a-d illustrate the insertion of the heating element 12 into an eye 32, according to an embodiment. Prior to the procedure, the loop 23 of the heating element 12 may be withdrawn into the insertion sleeve 19, so that, as seen in FIG. 3a, the loop 23 of heating element 12 is contained almost entirely within the insertion sleeve 19. Thus, the leading tip of the apparatus may be inserted into the anterior chamber 34 of the eye 32, as shown in FIG. 3a, through a small incision 505 (see FIG. 5).

As shown in FIG. 3b, the insertion sleeve 19 and collapsed heating element 12 may be pushed inside the lens capsule 36 (for posterior capsulotomy) (or near the anterior lens capsule for anterior capsulotomy). The loop 23 of the heating element 12 may then regain its pre-determined shape, as shown in FIG. 3c, and may then be positioned against the capsule 36. The transitional neck may not be perceptible from the top down perspective of the capsularhexis devices in FIGs. 3a-d. The heating element 12 may then be energized, e.g., with a short pulse or series of pulses of current. As discussed above, this heating may sear capsule 36 (e.g., the anterior lens capsule 509 and/or posterior lens capsule 513) to create a smooth continuous cut on the capsule 36. The heating element 12 may then be retracted into the insertion sleeve 19, as shown in FIG. 3d, and then removed from the eye 32. The cut portion of the capsule 36 may be readily removed using a conventional surgical instrument, such as forceps.

Because the superelastic wire 14 is flexible, the insertion sleeve 19 may be bent upwards when the heating element 12 is placed against the capsule 36. Because the deformation properties of the wire 14 (and, in some cases, the insulation 17) may be determined for a given device 10, the bending angle formed with respect to the plane of the heating element 12 may be used as an indication of the force applied to the capsule 36 by the heating element 12. Thus, a range of acceptable bending angles may be defined for a particular device 10, to correspond to a range of desirable application forces for optimal cauterization of the capsule 36. Accordingly, a surgeon may conveniently achieve a desired contact force between the heating element 12 and the capsule 36 by simply manipulating the bending angle to match or approximately match a pre-determined angle θ, as shown in FIG. 4. In some embodiments, angle θ may be defined as the angle between a plane of the loop 23 and the insulating portion 17 (which may be straight relative to the heating element 12 of the loop 23). For example, the angle θ may be characterized by the bend in the transitions between the loop 23 and the neck 21.

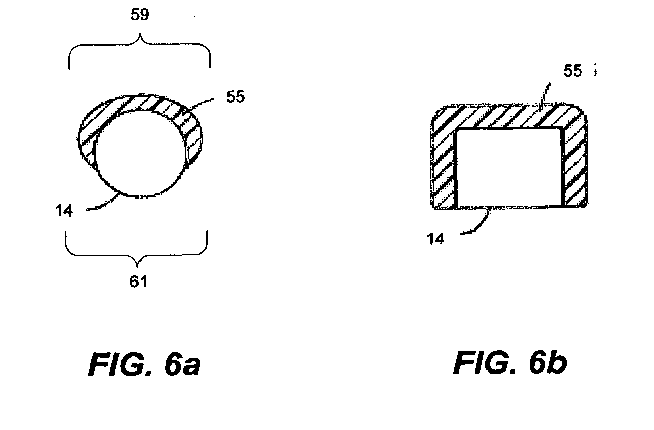

In some embodiments, to further reduce any potential collateral damage to tissue near the heating element 12, a thermally insulating layer may be disposed on at least a top face 59 of the loop 23 formed by the resistive-heating element 12, such that a bottom face 61, which may be disposed against the capsule 36 during the capsularhexis procedure, may be left bare. A cross-sectional view of one such embodiment is shown in FIG. 6A, which shows a cross-section of a round wire 14, partially surrounded with a thermally insulating layer 55. In some embodiments, the superelastic wire 14 may have a square or rectangular cross-section, as shown in FIG. 6B, in which case insulation 55 may be disposed on three sides of the wire 14. In either case, insulation 55 may be disposed on the wire 14 around all or substantially all of the loop 23 of the resistive-heating element 12.

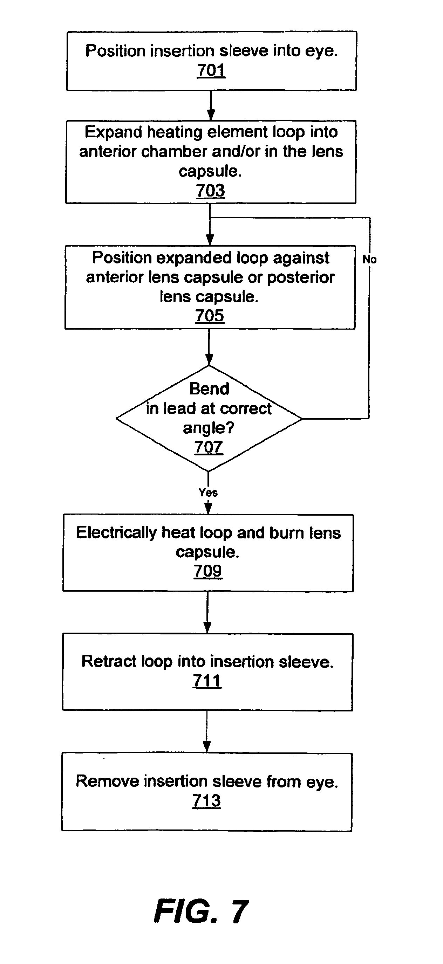

With the above-described device configurations in mind, those skilled in the art will appreciate that FIG. 7 illustrates a method for utilizing a capsularhexis device according to some embodiments, the method not being part of the invention. The elements provided in the flowchart are illustrative only. Various provided elements may be omitted, additional elements may be added, and/or various elements may be performed in a different order than provided below.

At 701, the insertion sleeve 19 may be positioned into the eye 32. The heating element 12 may be retracted into the insertion sleeve 19 prior to insertion into the eye. For example, the heating element 12 may be retracted by a surgeon and/or during manufacturing of the device 10. FIG. 1b illustrates an embodiment of a retracted heating element 12. In some embodiments, positioning the insertion sleeve 19 into the eye may include making a small incision 505 in the cornea 511 (or other part of the eye 32) for inserting the insertion sleeve 19.

At 703, the heating element loop 23 may be expanded into the anterior chamber 34 of the eye 32 (for anterior capsulorhexis) or in the lens capsule (for posterior capsulorhexis). Because the heating element 12 described herein may be collapsed, the insertion sleeve 19 may be dimensioned to fit through an incision 505 that is smaller than the expanded diameter 401 of the heating element's loop 23.

At 705, once the loop 23 of the heating element 12 is expanded into the eye 32, it may be positioned against the anterior lens capsule 509 and/or the posterior lens capsule 513. In some embodiments, the applied force between the heating element 12 and the capsule 36 may be gauged by assessing a bend in the lead section of the heating element 12.

At 707, the angle between the insertion sleeve 19 and the plane formed by the heating element 12 may be matched to a pre-determined angle (e.g., see FIG. 4) to determine if the correct force is applied.

At 709, after the heating element 12 is positioned against the capsule 36, the heating element 12 may be energized by the application of electrical current, so that the loop 23 may be heated to "burn" the lens capsule 36 with a substantially circular, continuous cut on the anterior lens capsule 509 and/or the posterior lens capsule 513.

At 711, once the burning of the capsule 36 is complete, the heating element 12 may be retracted into the insertion sleeve 19 and, at 713, the insertion sleeve 19 may be removed from the eye 32. In some embodiments, the detached portion of the capsule may be removed using a surgical instrument such as forceps.

As was briefly discussed above, the energizing of the resistance-heating element 12 may advantageously include a short pulse (e.g., 20 milliseconds) of electrical current, or a series of pulses (e.g., 1 millisecond each). In some embodiments, pulsed radiofrequency power may be used to reduce collateral thermal damage on the capsule and avoid electrochemical reaction at the gap 25. The frequency, waveform, voltage, pulse width, and duration of the radiofrequency power may be configured to attain a continuous through-cut on the capsule 36 while reducing collateral damage. Those skilled in the art will appreciate that the power settings (e.g., voltage, current, pulse width, number of pulses, etc.) may be established for a particular heating element configuration so that a continuous, circular (or oval) through-cut on the capsule 36 may be attained, while minimizing collateral damage to portions of the capsule 36 surrounding the portion to be removed. When determining the power settings for a particular heating element 12 according to those described herein, those skilled in the art may consider that multiple working mechanisms may contribute to the "cutting" of the capsule 36. For instance, a steam "explosion" in the viscoelastic material and tissue water caused by rapid heating of the heating element 12 may contribute to the cut-through of the capsule 36, in addition to the thermal breakdown of the capsule material.

In some embodiments, the capsularhexis device 10 and/or a management system for the capsularhexis device 10 (e.g., handpiece 41 and/or console 43) may include one or more processors (e.g., processor 1001) and/or memories 1003. The processor 1001 may include single processing devices or a plurality of processing devices. Such a processing device may be a microprocessor, controller (which may be a micro-controller), digital signal processor, microcomputer, central processing unit, field programmable gate array, programmable logic device, state machine, logic circuitry, control circuitry, analog circuitry, digital circuitry, and/or any device that manipulates signals (analog and/or digital) based on operational instructions. The memory 1003 coupled to and/or embedded in the processors 1001 may be a single memory device or a plurality of memory devices. Such a memory device may be a read-only memory, random access memory, volatile memory, non-volatile memory, static memory, dynamic memory, flash memory, cache memory, and/or any device that stores digital information. Note that when the processors 1001 implement one or more of its functions via a state machine, analog circuitry, digital circuitry, and/or logic circuitry, the memory 1003 storing the corresponding operational instructions may be embedded within, or external to, the circuitry comprising the state machine, analog circuitry, digital circuitry, and/or logic circuitry. The memory 1003 may store, and the processor 1001 may execute, operational instructions corresponding to at least some of the elements illustrated and described in association with FIG. 7.

Various modifications may be made to the presented embodiments by a person of ordinary skill in the art. For example, although some of the embodiments are described above in connection with capsularhexis devices 10 it can also be used with other thermal cutting surgical devices. Other embodiments of the present invention will be apparent to those skilled in the art from consideration of the present specification and practice of the present invention disclosed herein. It is intended that the present specification and examples be considered as exemplary only with a true scope of the invention being indicated by the following claims and equivalents thereof.

## Claims

1. A capsularhexis device (10), comprising:
a resistive-heating element (12) comprising an electrically resistive, superelastic wire having first and second ends, the superelastic wire forming a loop (23) with a gap between the first and second ends (31); and
an insulating portion (17) comprising an electrically insulating material separating the first and second ends of the superelastic wire, wherein the first and second ends are adjacent to each other and at least partially extend at an angle from a planar face defined by the loop, to the insulating portion, to form a transitional neck (21) between the loop and the insulating portion;
wherein a gap between the first and second ends at the insulating portion, on one side of the transitional neck is wider than a gap between the first and second ends on an opposing side of the transitional neck at the loop.

2. The capsularhexis device of claim 1, wherein at least partially extending at an angle from the planar face defined by the loop (23) comprises extending approximately perpendicular from the planar face defined by the loop.

3. The capsularhexis device of claim 1, wherein at least partially extending at an angle from the planar face defined by the loop (23) comprises extending approximately 45 degrees as measured to a back side of the planar face defined by the loop.

4. The capsularhexis device of claim 1, further comprising an insertion sleeve (19) configured to fit around the insulating portion (17) and adapted to substantially contain the resistive-heating element (12) when the resistive-heating element is in a retracted position.

5. The capsularhexis device of claim 1, wherein the superelastic wire is formed from a nickel titanium alloy.

6. The capsularhexis device of claim 1, wherein the loop (23) has a bottom face adapted, for placing against an anterior lens capsule or posterior lens capsule of an eye, and a top face, opposite the bottom face, and wherein the resistive-heating element (12) further comprises a thermally insulating layer (55) disposed on at least the top face but absent from the bottom face.

7. The capsularhexis device of claim 1, wherein the superelastic wire has a rectangular cross section around at least substantially the entire loop (23), and wherein the thermally insulating layer (55) is disposed on three sides of the superelastic wire around at least substantially the entire loop.

8. The capsularhexis device of claim 1, wherein the gap (25) between the first and second ends on the opposing side of the transitional neck (21) is approximately 0.0762mm [0.003 inches].

9. The capsularhexis device of claim 1, wherein a diameter of the loop (23) is approximately 2-4 millimeters to allow the loop to be used for a posterior capsulotomy.

10. The capsularhexis device of claim 1, wherein a diameter of the loop (23) is approximately 4-6 millimeters to allow the loop to be used for an anterior capsulotomy.

## Patentansprüche

1. Kapsulorhexis-Vorrichtung (10), aufweisend:
ein Widerstands-Heizelement (12) aufweisend einen elektrisch widerständigen superelastischen Draht mit einem ersten und zweiten Ende, wobei der superelastische Draht eine Schleife (23) mit einem Zwischenraum zwischen dem ersten und zweiten Ende (31) bildet; und
einen Isolationsabschnitt (17), der ein elektrisch isolierendes Material aufweist, mit dem das erste und zweite Ende des superelastischen Drahtes separiert sind, wobei das erste und zweite Ende einander angrenzen und sich zumindest teilweise in einem Winkel von einer durch die Schleife definierten planaren Stirnfläche zu dem Isolationsabschnitt erstrecken, um einen Übergangshals (21) zwischen der Schleife und dem Isolationsabschnitt zu bilden; wobei ein Zwischenraum zwischen dem ersten und zweiten Ende an dem Isolationsabschnitt auf einer Seite des Übergangshalses breiter ist als ein Zwischenraum zwischen dem ersten und zweiten Ende auf einer gegenüberliegenden Seite des Übergangshalses an der Schleife.

2. Kapsulorhexis-Vorrichtung (10) nach Anspruch 1, wobei sich die zumindest teilweise in einem Winkel von durch die Schleife (23) definierte planare Stirnflächenerstreckung eine Erstreckung von näherungsweise senkrechter Art von der durch die Schleife definierten planaren Stirnfläche umfasst.

3. Kapsulorhexis-Vorrichtung nach Anspruch 1, wobei die sich zumindest teilweise in einem Winkel von der durch die Schleife (23) definierte planare Stirnflächenerstreckung eine Erstreckung von näherungsweise 45 Grad, gemessen an eine Rückseite der durch die Schleife definierten planaren Stirnfläche aufweist.

4. Kapsulorhexis-Vorrichtung nach Anspruch 1, ferner aufweisend einen Einsatz-Überwurf (19), der um den Isolationsabschnitt (17) passt und dazu bestimmt ist, das Widerstands-Heiz-Element (12) im Wesentlichen aufzunehmen, wenn das Widerstands-Heiz-Element in einer zurückgezogenen Position vorliegt.

5. Kapsulorhexis-Vorrichtung nach Anspruch 1, wobei der superelastische Draht aus einer Nickel-Titan-Legierung gebildet ist.

6. Kapsulorhexis-Vorrichtung nach Anspruch 1, wobei die Schleife (23) eine Bodenfläche hat, die dazu bestimmt ist, gegen eine vordere Linsenkapsel oder eine hintere Linsenkapsel eines Auges platziert zu werden, sowie eine obere Stirnfläche gegenüber der Bodenfläche, und wobei das Widerstands-Heiz-Element (12) ferner eine thermisch isolierte Schicht (55) aufweist, die auf zumindest der oberen Stirnfläche angeordnet jedoch absent von der Bodenfläche ist.

7. Kapsulorhexis-Vorrichtung nach Anspruch 1, wobei der superelastische Draht einen rechtwinkligen Querschnitt um zumindest im Wesentlichen die gesamte Schleife (23) hat, und wobei die thermisch isolierende Schicht (55) auf drei Seiten des superelastischen Drahtes um zumindest im Wesentlichen die gesamte Schleife angeordnet ist.

8. Kapsulorhexis-Vorrichtung nach Anspruch 1, wobei der Zwischenraum (25) zwischen dem ersten und zweiten Ende auf der gegenüberliegenden Seite des Übergangshalses (21) näherungsweise 0,0762 mm (0,003 inch) ist.

9. Kapsulorhexis-Vorrichtung nach Anspruch 1, wobei ein Durchmesser der Schleife (23) näherungsweise 2-4 mm ist, um der Schleife zu ermöglichen, für eine hintere Kapsulotomie verwendet zu werden.

10. Kapsulorhexis-Vorrichtung nach Anspruch 1, wobei der Durchmesser der Schleife (23) näherungsweise 4 - 6 mm ist, um der Schleife zu ermöglichen, für eine vordere Kapsulotomie verwendet zu werden.

## Revendications

1. Dispositif de capsulorhexis (10), comprenant :
un élément chauffant résistif (12) comprenant un fil super-élastique électriquement résistif, ayant des première et seconde extrémités, le fil super-élastique formant une boucle (23) avec un espace entre les première et seconde extrémités (31) ; et
une partie isolante (17) comprenant un matériau électriquement isolant séparant les première et seconde extrémités du fil super-élastique, dans lequel les première et seconde extrémités sont adjacentes entre elles et s'étendent au moins partiellement selon un angle, à partir d'une face plane définie par la boucle et jusqu'à la partie isolante, afin de former un col de transition (21) entre la boucle et la partie isolante ;
dans lequel un espace entre les première et seconde extrémités au niveau de la partie isolante, sur un côté du col de transition, est plus large qu'un espace entre les première et seconde extrémités sur un côté opposé du col de transition au niveau de la boucle.

2. Dispositif de capsulorhexis selon la revendication 1, dans lequel l'extension au moins partielle selon un angle à partir de la face plane définie par la boucle (23) comprend l'extension approximativement perpendiculaire à partir de la face plane définie par la boucle.

3. Dispositif de capsulorhexis selon la revendication 1, dans lequel l'extension au moins partielle selon un angle à partir de la face plane définie par la boucle (23) comprend l'extension approximativement à 45 degrés telle que mesurée sur un côté arrière de la face plane définie par la boucle.

4. Dispositif de capsulorhexis selon la revendication 1, comprenant en outre un manchon d'insertion (19) configuré pour s'adapter autour de la partie isolante (17) et adapté pour sensiblement contenir l'élément chauffant résistif (12) lorsque l'élément chauffant résistif est dans une position rétractée.

5. Dispositif de capsulorhexis selon la revendication 1, dans lequel le fil super-élastique est formé à partir d'un alliage de nickel-titane.

6. Dispositif de capsulorhexis selon la revendication 1, dans lequel la boucle (23) a une face inférieure adaptée pour un placement contre une capsule antérieure du cristallin ou une capsule postérieure du cristallin d'un oeil, et une face supérieure, opposée à la face inférieure, et dans lequel l'élément de chauffage résistif (12) comprend en outre une couche thermiquement isolante (55) disposée au moins sur la face supérieure mais absente de la face inférieure.

7. Dispositif de capsulorhexis selon la revendication 1, dans lequel le fil super-élastique a une section transversale rectangulaire autour d'au moins sensiblement la totalité de la boucle (23), et dans lequel la couche thermiquement isolante (55) est disposée sur trois côtés du fil super-élastique autour d'au moins sensiblement la totalité de la boucle.

8. Dispositif de capsulorhexis selon la revendication 1, dans lequel l'espace (25) entre les première et seconde extrémités sur le côté opposé du col de transition (21) est d'approximativement 0,0762 mm [0,003 pouce].

9. Dispositif de capsulorhexis selon la revendication 1, dans lequel un diamètre de la boucle (23) est d'approximativement 2-4 millimètres pour permettre d'utiliser la boucle pour une capsulotomie postérieure.

10. Dispositif de capsulorhexis selon la revendication 1, dans lequel un diamètre de la boucle (23) est d'approximativement 4-6 millimètres pour permettre d'utiliser la boucle pour une capsulotomie antérieure.
